# FASCICULE DE BREVET EUROPEEN

(11) **EP 3 428 249 B1**
(45) Date de publication et mention de la délivrance du brevet: **18.12.2019**
(21) Numéro de dépôt: 18180989.8
(22) Date de dépôt: 29.06.2018
(51) Int. Cl.: C10G 50/00, C10G 53/04, C10G 53/06, C10G 55/06, C10G 57/02, C10G 70/04, C10G 70/06, C10G 11/18, C10G 21/00

(54) **METHODE ET PROCEDE POUR CONVERTIR L'ETHYLENE PRESENT DANS L'EFFLUENT DE TETE D'UN FCC DE MANIERE A AUGMENTER LA PRODUCTION DE PROPYLENE**
METHODE UND VERFAHREN ZUM UMWANDELN VON IN EINEM ABFLUSS EINES FCC-BESTÜCKKOPFS ENTHALTENEM ETHYLEN ZUR ERHÖHUNG DER PROPYLEN-PRODUKTION
METHOD AND PROCESS FOR CONVERTING ETHYLENE PRESENT IN FCC HEAD EFFLUENT SO AS TO INCREASE THE PRODUCTION OF PROPYLENE

(30) Priorité: 13.07.2017 FR 1756712
(43) Date de publication de la demande: 16.01.2019
(73) Titulaire: IFP Energies nouvelles, 92852 Rueil-Malmaison Cedex (FR)
(72) Inventeur: FISCHER, Beatrice, 69005 LYON (FR); COUPARD, Vincent, 69100 VILLEURBANNE (FR)
(74) Mandataire: IFP Energies nouvelles

(56) Documents cités:
- US-A1- 2007 185 359
- US-A1- 2011 243 797
- Margaret M Shreehan: "Recovery of olefins from refinery offgases", digitalrefining, 1 octobre 1998 (1998-10-01), pages 1-9, XP055513475, Extrait de l'Internet: URL:http://www.digitalrefining.com/article _1000215.pdf [extrait le 2018-10-08]
- JUN JONG-WON ET AL: "Conversion of Y into SSZ-13 zeolites and ethylene-to-propylene reactions over the obtained SSZ-13 zeolites", CHEMICAL ENGINEERING JOURNAL, ELSEVIER SEQUOIA, LAUSANNE, CH, vol. 303, 8 juin 2016 (2016-06-08), pages 667-674, XP029708618, ISSN: 1385-8947, DOI: 10.1016/J.CEJ.2016.06.043
- AARON AKAH ET AL: "Maximizing propylene production via FCC technology", APPLIED PETROCHEMICAL RESEARCH, vol. 5, no. 4, 22 mars 2015 (2015-03-22), pages 377-392, XP055405038, ISSN: 2190-5525, DOI: 10.1007/s13203-015-0104-3

## Description

Dans une unité de craquage catalytique (communément appelée FCC), à la sortie de l'ensemble réaction/régénération, se situe une colonne de fractionnement, qui permet de séparer les fractions lourdes, le naphta lourd, et les fractions légères : gaz, GPL et essence légères qui se retrouvent en tête de colonne. Ces fractions légères de tête sont ensuite envoyées à une section permettant de récupérer un maximum de GPL et d'essence, et de purifier éventuellement le gaz avant de l'envoyer au gaz combustible. Ce gaz, dit « gaz combustible », contient une quantité non négligeable d'éthylène (noté quelquefois C2--), qui est souvent brûlé avec le gaz combustible. On peut trouver une référence assez complète dans : "Fluid Catalytic Cracking Handbook" - Reza Sadeghbeigi - 3eme édition - chapitre 1.

Dans de rares cas, le gaz combustible est purifié davantage au moyen d'un fractionnement cryogénique permettant de récupérer l'éthylène. Cette option est coûteuse, et ne se justifie que pour des unités importantes, avec de gros débits d'éthylène. Les composés les plus recherchés en pratique sont le propylène (noté quelquefois C3--), et l'essence, et de nombreux brevets existent pour tenter d'augmenter leur production. Il existe une tendance actuelle visant à augmenter le rendement du FCC en propylène : le rendement habituel est inférieur à 5%, mais en changeant le type de charge et les conditions opératoires, on peut avoir jusqu'à 18% de propylène par rapport à la charge. La quantité d'éthylène produite est également augmentée, mais comme l'éthylène sort sous forme gazeuse, très dilué par l'azote, le dioxyde de carbone, l'hydrogène, le méthane, l'éthane, d'autres alcanes, d'autres oléfines, et par d'autres impuretés (CO, H2S, NH3, S02, N02, etc.), il est très difficile à récupérer, et il est envoyé généralement vers le gaz combustible de la raffinerie.

US20110243797 divulgue un dispositif et procédé pour le fractionnement de la fraction gazeuse d'une unité de craquage catalytique pour convertir l'éthylène en dimers et trimers valorisables.

### DESCRIPTION SOMMAIRE DES FIGURES

La figure 1 selon l'art antérieur, représente le schéma de fractionnement des effluents d'une unité de craquage catalytique (FCC) limité à la partie légère desdits effluents, c'est-à-dire à l'essence légère, au GPL et à un gaz résiduel dit « gaz combustible » car, selon l'art antérieur, ce gaz est le plus souvent envoyé au réseau combustible de la raffinerie sans séparation ultérieure.
La figure 2 représente le schéma de base du procédé selon l'invention, dans lequel le gaz combustible est envoyé dans une unité de conversion de l'éthylène en propylène (22). Le schéma est complété par une section d'absorption (31) et une section de débutanisation (35).
La figure 3 représente une variante du schéma selon l'invention, dans laquelle la conversion de l'éthylène dans l'unité de conversion (22) n'étant pas totale, il peut être avantageux de recycler une partie du gaz sortant de l'unité de conversion (22), après compression par le compresseur (26), vers la section de conversion (22) au moyen du conduit (38).
La figure 4 représente de manière plus détaillée l'unité (22) de conversion de l'éthylène en propylène selon l'invention. La réaction principale étant très exothermique, il est nécessaire d'avoir au moins deux réacteurs (223) et (227) avec un refroidissement intermédiaire (225).
La figure 5 représente une variante du l'unité de conversion (22) selon l'invention dans laquelle la charge de l'unité (22) de conversion de l'éthylène en propylène comporte une forte proportion d'effluent recyclé (222), ce qui permet à l'unité de fonctionner avec un seul réacteur (223).

### DESCRIPTION SOMMAIRE DE L'INVENTION

La présente invention consiste en un procédé permettant de transformer en propylène et autres produits récupérables l'éthylène contenu dans le gaz combustible, c'est-à-dire le gaz de tête issu du fractionnement des effluents d'une unité de craquage catalytique, après séparation de la fraction essence légère et des GPL. Le gaz de tête issu du fractionnement des effluents d'une unité FCC contient en effet une fraction essence légère, les GPL (gaz de pétrole liquéfiés), et un gaz résiduel qui est généralement utilisé comme combustible. Or ce gaz combustible contient une certaine proportion d'éthylène qu'il est possible de valoriser, ceci au moyen d'une réaction de transformation de l'éthylène principalement en propylène et autres produits d'intérêt qui nécessite une unité de conversion catalytique qui s'incorpore au schéma de traitement des effluents de tête selon l'art antérieur.

L'objet de la présente invention est donc de récupérer la plus grande partie de l'éthylène contenu dans le gaz combustible, pour le convertir principalement en propylène. D'autres produits valorisables, essentiellement le butène et des aromatiques sont également produits. Ces produits seront récupérés respectivement dans le GPL et dans la coupe essence.

L'unité de conversion de l'éthylène en propylène est intégrée au schéma de fractionnement des effluents de tête du FCC, qu'on appelle plus précisément section de récupération pour traiter les effluents légers sortant en tête du fractionnement principal.

Le catalyseur utilisé dans l'unité de conversion de l'éthylène en propylène comprend une zéolite qui, à haute température (entre 500 et 650°C), permet d'opérer sans se désactiver da façon trop rapide, malgré les impuretés non hydrocarbonées présentes avec l'éthylène de charge. Le catalyseur préféré est un catalyseur comprenant de la ZSM-5 dans une matrice inerte, par exemple la silice. Des post traitement peuvent être réalisé sur ce catalyseur pour limiter sa désactivation en cycle. Toute zéolithe permettant de réaliser des réactions d'interconversion d'oléfines (c'est-à-dire de conversion entre oléfines, comme le fait l'unité de conversion (22)) sera adaptée à ce procédé. La conversion d'éthylène en propylène est fortement exothermique, mais comme l'éthylène est très dilué, et que d'autres réactions portant sur d'autres oléfines présentes dans le mélange sont endothermiques, cette forte exothermicité n'est pas un problème, et il est possible dans beaucoup de cas d'utiliser un seul réacteur. On utilise généralement l'exothermicité de la réaction de conversion de C2-- en C3-- pour réaliser une préchauffe de la charge au moyen d'un échangeur charge effluent.

Le ou les réacteurs utilisés pour la réaction de conversion de C2-- en C3-- sont des réacteur ascendant ou descendant ou radiaux, c'est-à-dire à écoulement transverse de la charge au travers du lit catalytique. Le lit catalytique peut être du type lit fixe ou en lit mobile. Un lit mobile est un lit à écoulement gravitaire du type de celui qu'on rencontre dans les unités de reformage catalytique des essences.

Le ou les réacteurs sont également couplés avec une boucle de régénération par combustion permettant d'opérer le procédé en continu. La réaction de conversion nécessite une pression partielle d'oléfines comprise entre 1 et 2 bars (et de préférence entre 1,25 et 1,75 bar), mais grâce à la forte dilution, on peut opérer à une pression comprise entre 6 et 12 bar suivant les cas, c'est-à-dire une pression pas trop éloignée de la pression de la section de récupération (15-20 bar).

La régénération est opérée à la même température que l'opération de réaction, et de préférence à la même pression, ce qui permet de passer rapidement un réacteur de réaction en régénération, et de régénération en réaction. Le réacteur basculé en régénération (pendant que le réacteur régénéré est basculé en réaction) est connecté à une section de régénération comprenant un échangeur charge/effluent, un échangeur de réfrigération, un séparateur permettant de condenser l'eau de réaction, et un compresseur de recirculation du gaz de régénération.

La régénération se fait par brûlage du coke déposé sur le catalyseur en quelques heures, en injectant dans le gaz de régénération la quantité d'air adaptée, éventuellement diluée avec un inerte, par exemple de l'azote ou les gaz de combustion appauvris en oxygène, pour avoir une augmentation de température dans le réacteur limitée au maximum à 50°C, ce qui permet de se passer de four de régénération. Le nombre de réacteurs est adapté afin d'avoir toujours un réacteur au moins en réaction et un réacteur en régénération.

Le positionnement de l'unité de conversion entre la première et la deuxième section d'absorption généralement déjà existantes dans un schéma de fractionnement selon l'art antérieur, permet de remodeler un FCC aisément et à moindre coût, sans changer les équipements principaux existants. La production supplémentaire de propylène est de l'ordre de 10%, ce qui permet aux unités aval (colonne dite de « superfractionnement » propane propylène en particulier) d'absorber cette production supplémentaire avec des modifications mineures, ou même sans aucune modification si les marges de dimensionnement sont suffisantes.

Plus précisément, la présente invention consiste en un procédé de fractionnement de la fraction gazeuse sortant en tête de la colonne de fractionnement d'une unité de craquage catalytique (FCC), fraction contenant les GPL (abréviation de gaz de pétrole liquéfié), l'essence légère, et un gaz résiduel dit « gaz combustible » qui contient lui-même une certaine quantité d'éthylène. C'est cet éthylène que la présente invention cherche à valoriser en propylène et autres produits d'intérêt.

Le procédé selon l'invention comprend généralement :
- une première section d'absorption (6) permettant de séparer le gaz combustible (15) et le flux d'essence légère et de GPL (10), cette section étant généralement existante dans un FCC, et ne nécessitant aucune modification par suite de l'installation des équipements selon la présente invention (l'unité de conversion (22), et les sections d'absorption (31) et de débutanisation (35)).
- une unité de conversion (22) de l'éthylène en propylène, dans laquelle une grande partie de l'éthylène est convertie essentiellement en propylène, en aromatiques et autres produits d'intérêt, les autres oléfines présentes (C4, C5+) étant également partiellement converties, essentiellement en propylène,
- une nouvelle section d'absorption (31) qui admet les effluents de l'unité de conversion (22) après séparation dans le séparateur (24) produisant une phase gaz (25) qui est recomprimé dans le compresseur (26) avant d'entrer dans la nouvelle section d'absorption (31), et une phase liquide (28) qui est pompée au moyen de la pompe (29) pour entrer également dans la nouvelle section d'absorption (31). Le propylène, le butène, les fractions essences contenues dans la fraction gaz (27) et la fraction liquide (30), sont récupérés dans ladite section d'absorption (31), en utilisant de l'essence (32) arrivant du débutaniseur existant (11),
- une seconde section d'absorption (16) qui admet le gaz (33) issu de la nouvelle section d'absorption. Cette seconde section d'absorption (16) existe généralement dans un schéma de FCC classique, et ne nécessite aucune modification qui pourraient être induites par l'installation des équipements selon la présente invention (c'est-à-dire la section d'absorption (31) présentée plus haut, et l'unité de conversion (22), et la section de débutanisation (35) décrites ci-dessous).
- une nouvelle section de débutanisation (35) qui admet les fractions GPL et essence (34) issus de la nouvelle section d'absorption (31) et qui produit une essence stabilisée (36) et une fraction légère (37) qui peut être mélangé avec le GPL produit.

L'unité de conversion (22) de l'éthylène en propylène et autres produits d'intérêt est une unité catalytique utilisant un catalyseur à base de zéolite travaillant à une température comprise entre 500°C et 650°C, et sous une pression partielle d'oléfines comprise entre 1 et 2 bars.

Selon une première variante du procédé de fractionnement selon la présente invention, une partie du flux gazeux (27) issu de l'unité de conversion de l'éthylène en propylène et autres produits d'intérêt (23) est recyclé à l'entrée de l'unité de conversion après compression dans le compresseur (26), ladite fraction recyclée pouvant varier entre 10% et 75 % du flux total (27) arrivant à la section (31).

Dans le procédé de fractionnement selon la présente invention, l'unité de conversion (22) de l'éthylène en propylène et autres produits d'intérêt comprend au moins deux réacteurs (223) et (227) séparés par un refroidissement intermédiaire de l'effluent (224) du premier réacteur (223) allant vers le second réacteur (227), au moyen d'un premier échangeur (225), puis en sortie de réacteur (227), l'effluent converti (228) est envoyé vers l'échangeur charge-effluent (221), où il est refroidi par échange indirect avec la charge (15), puis vers le réfrigérant (230), où le refroidissement est terminé à l'aide d'eau de refroidissement.

Selon une autre variante du procédé de fractionnement selon la présente invention, la charge (15) de l'unité de conversion (22) arrive en mélange avec le flux recyclé (38) issu d'une partie du flux (27) vers l'échangeur charge effluent (221), où les deux flux mélangés sont préchauffés jusqu'à la température d'opération, puis envoyé vers le réacteur (223) de l'unité de conversion (22), l'effluent converti (228) étant envoyé vers l'échangeur charge-effluent (221), dans lequel il est refroidi par échange indirect avec la charge (15), puis vers le réfrigérant (230), dans lequel le refroidissement est terminé à l'aide d'eau de refroidissement, l'effluent refroidi (23) étant envoyé vers un séparateur (24), dans lequel la phase liquide est séparée de la phase gazeuse, ladite phase gazeuse (25) étant comprimée par le compresseur (26) puis envoyée vers la section d'absorption (31), et le liquide (28), étant envoyé vers la section d'absorption (31) par la pompe (29).

Selon une variante préférée du procédé de fractionnement selon la présente invention, l'unité de conversion de l'éthylène en propylène et autres produits d'intérêt (22) fonctionne avec un catalyseur à base de zéolite aux conditions opératoires suivantes :
- température comprise entre 500°C et 650°C,
- pression partielle d'éthylène comprise entre 1 et 2 bar absolus.

### DESCRIPTION DETAILLEE DE L'INVENTION

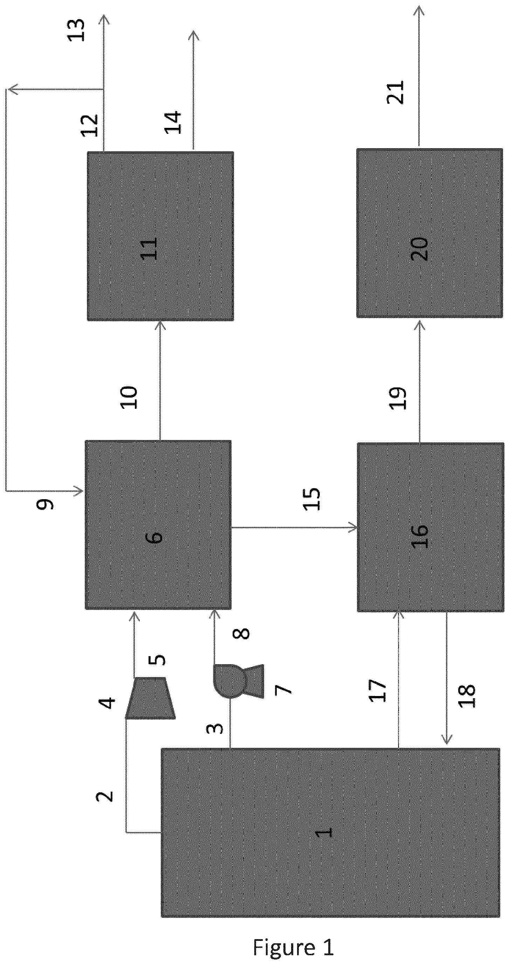

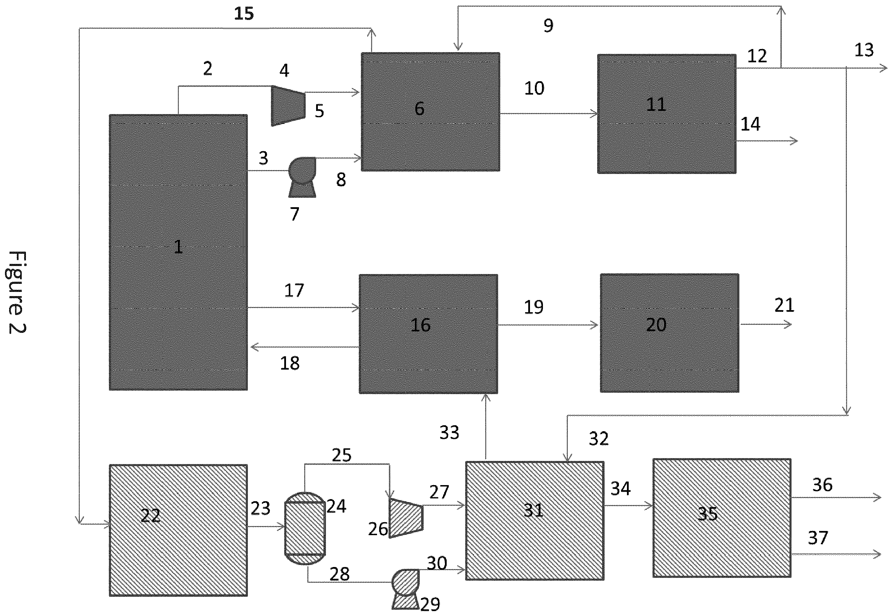

La présente invention sera mieux comprise au moyen de la description des figures qui l'illustrent. La figure 1 étant selon l'art antérieur, et les figures 2, 3, 4 et 5 selon la présente invention.

Description de la figure 1 (selon l'art antérieur) : fractionnement des fractions légères sortie FCC. De la colonne de fractionnement principal du FCC (1) sortent en tête un distillat vapeur (2) et un distillat liquide (3). Ces deux fractions comprennent essentiellement des hydrocarbures légers, (paraffines et oléfines), depuis le méthane jusqu'à l'essence légère, de l'hydrogène, de l'azote, des oxydes de carbone (CO et CO2), de l'eau, et de petites quantités d'H₂S et de NH₃, ainsi que des traces de SOx et NOx. La fraction vapeur est comprimée par le compresseur (4) et envoyée par le conduit (5) vers une première section d'absorption (6).

La fraction liquide est pompée par la pompe (7) et envoyée par le conduit (8) vers la section d'absorption (6). Cette section utilise une essence d'absorption arrivant par le conduit (9) pour récupérer l'essentiel des fractions liquides, qui sont envoyées par le conduit (10) vers une section de débutanisation (11) où sont séparés d'une part l'essence sortant par le conduit (12), et les GPL (C3/C4) sortant par le conduit (14). Une partie de l'essence est renvoyée par le conduit (9) vers la section (6), et le reste constitue le produit essence, envoyé par le conduit (13) à l'extérieur de l'unité.

De la section (6) sort également par le conduit (15) la fraction gazeuse qui a perdu l'essentiel des GPL et de l'essence. Pour finir de récupérer le reste de produits valorisable, ce gaz est envoyé à une deuxième section d'absorption (16) ou une coupe lourde venant de la colonne de fractionnement (1) par le conduit (17) permet de renvoyer vers la colonne par le conduit (18) avec la coupe lourde une grande partie de ce reste de GPL et d'essence. Le gaz léger purifié est envoyé par le conduit (19) vers une section de purification (20) avant d'être envoyé vers le gaz combustible par le conduit (21).

Tout l'éthylène contenu dans le flux (21) est brûlé. Le récupérer nécessiterait une purification très poussée suivie d'une distillation cryogénique, ce qui supposerait des investissements souvent considérés comme trop importants par rapport à la quantité produite. L'objet de la présente invention est de transformer l'éthylène sans le purifier et de le convertir essentiellement en propylène et autres produits d'intérêt qui seront récupérés en partie dans l'unité existante. Ceci sera mieux compris au vu de la figure 2 qui représente un schéma du procédé suivant l'invention.

Description de la figure 2 (selon l'invention) : fractionnement des fractions légères sortie FCC, avec conversion de l'éthylène en propylène et autres produits d'intérêt.

La fraction gazeuse sortant de la section (6) par le conduit (15) n'est plus envoyée vers la deuxième section d'absorption (16), mais vers la nouvelle unité de conversion (22), dans laquelle une grande partie de l'éthylène est convertie essentiellement en propylène, en aromatiques, et autres produits d'intérêt. Les autres oléfines présentes (C4, C5+) sont également partiellement converties, essentiellement en propylène. A la sortie de l'unité (22), le gaz est à plus faible pression, et doit être recomprimé pour être envoyé à une section d'absorption. Il est donc envoyé par le conduit (23) vers un séparateur (24), la phase vapeur est envoyée par le conduit (25) vers un dispositif de compression (26) dont il ressort à plus forte pression par le conduit (27).

La phase liquide est envoyée par le conduit (28) vers la pompe (29), dont elle ressort à plus forte pression par le conduit (30) pour entrer dans la section d'absorption (31). Le propylène, le butène et les fractions essences contenues dans le gaz (25), sont récupérés dans la section d'absorption (31), en utilisant de l'essence arrivant par le conduit (32) du débutaniseur existant (11). Le gaz après absorption est envoyé par le conduit (33) à la section d'absorption secondaire existante (16), et les fractions GPL et essence sont envoyées par le conduit (34) vers la section (35) de débutanisation. L'essence stabilisée sort par le conduit (36), et pourra être envoyée au stockage en mélange avec l'essence sortant par le conduit (13). La fraction légère du débutaniseur sort par le conduit (37), et pourra être mélangé avec le GPL produit sortant par le conduit (14).

Il serait possible d'utiliser les sections existantes (6) et (11) au lieu d'utiliser de nouveaux équipements correspondant aux nouvelles sections (31) et (35). Cependant, les équipements des sections (6) et (11) risqueraient d'être insuffisants et nécessiteraient de grosses modifications, ce qui imposerait un arrêt de l'unité FCC pour plusieurs mois. L'avantage de créer de nouvelles sections (31) et (35) est de permettre de revamper un FCC existant sans grandes modifications, uniquement avec quelques connections vers la nouvelle unité, ce qui pourrait être fait en peu de temps lors d'un arrêt normal.

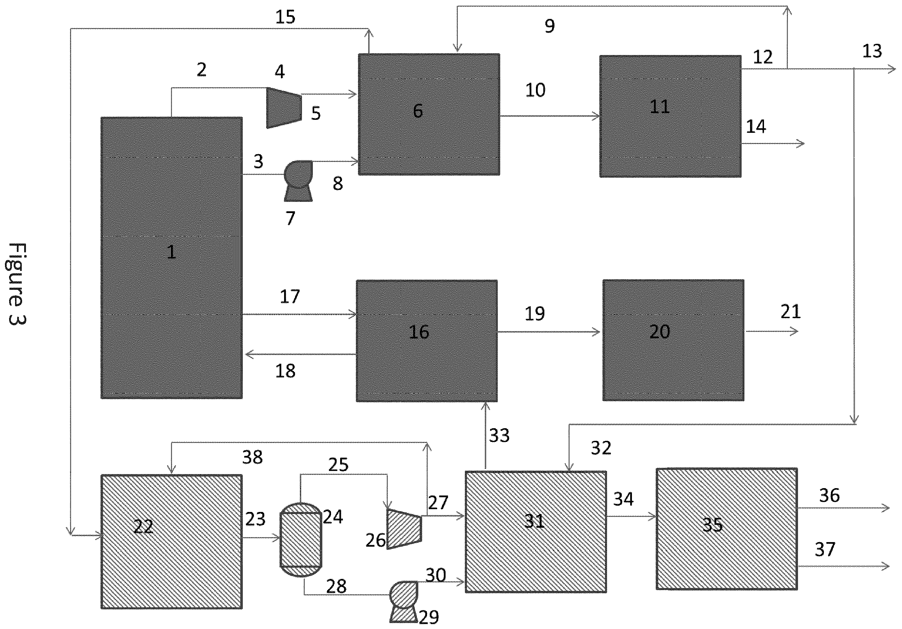

Description de la figure 3 (variante selon l'invention) : fractionnement des fractions légères sortie FCC avec conversion de l'éthylène en propylène et autres produits d'intérêt, et recyclage d'une partie du gaz sortant de l'unité de conversion.

La conversion de l'éthylène dans l'unité de conversion (22) n'étant pas totale, il peut être avantageux de recycler une partie du gaz sortant de l'unité de conversion (22), et comprimé par le compresseur (26), vers la section de conversion (22) par le conduit (38).

L'éthylène restant après conversion étant encore plus dilué que dans la charge (15), cela permet d'avoir une pression du réacteur de conversion plus élevée pour la même pression partielle d'oléfines, ce qui est favorable économiquement. De plus l'exothermicité de la réaction de conversion en propylène est diminuée, ce qui peut permettre d'opérer avec un seul réacteur, donc diminue le coût de l'unité, malgré sa capacité augmentée.

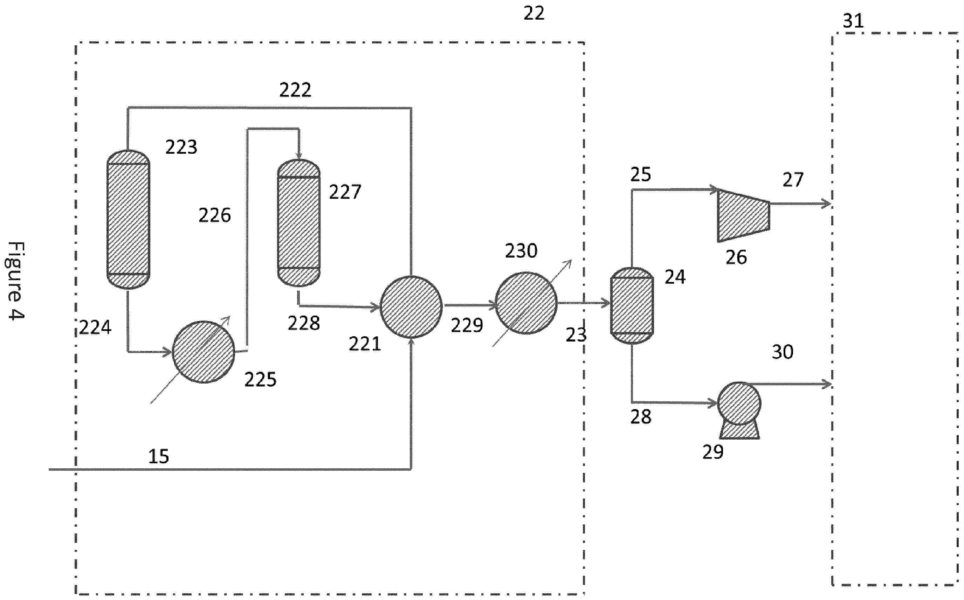

Description de la figure 4 (section de conversion selon l'invention) : unité de conversion d'éthylène à deux réacteurs avec refroidissement intermédiaire.

Le flux sortie de l'absorbeur existant (6) arrive par le conduit (15) vers l'échangeur charge -effluent (221) ou il est préchauffé jusqu'à la température d'opération et envoyé vers le premier réacteur (223). La réaction étant très exothermique, il est nécessaire de refroidir en sortie de ce premier réacteur dans la génération de vapeur (225) afin de refroidir l'effluent (224) jusqu'à la température d'opération. Le flux refroidi est envoyé par le conduit (226) vers le deuxième réacteur (227). Deux réacteurs (223) et (227) sont figurés sur ce schéma. Dans certains cas, il pourrait être nécessaire d'en installer davantage si la proportion d'éthylène en entrée, donc l'exothermicité de la réaction de conversion de l'éthylène en propylène est très grande. En sortie du second réacteur (227), l'effluent converti est envoyé par le conduit (228) vers l'échangeur charge-effluent (221), où il est refroidi par échange indirect avec la charge, puis vers le réfrigérant (230), dans lequel le refroidissement est terminé à l'aide d'eau de refroidissement. Il est également possible d'utiliser de l'air pour assurer le refroidissement au niveau du réfrigérant (230).

En sortie du réfrigérant (230) cet échangeur, l'effluent est envoyé par le conduit (23) vers un séparateur (24), ou la phase liquide est séparée de la phase gazeuse. La phase gazeuse est reprise dans le conduit (25), comprimée par le compresseur (26), et envoyée vers une section d'absorption par le conduit (27). Le liquide, soutiré par le conduit (28), est envoyée vers la même section d'absorption par la pompe (29) et le conduit (30).

Cette première variante nécessite en général un nombre de réacteurs (223) de 2, 3 ou 4 fonctionnant en série, plus un certain nombre fonctionnant en régénération pour avoir une opération continue, plus éventuellement encore un certain nombre « en réserve », au cas où la durée de vie du catalyseur serait inférieure à 1 an pour pouvoir changer le catalyseur en continuant d'opérer. De plus les pertes de charges dans tous ces réacteurs, auxquelles s'ajoute la perte de charge dans le ou les générateurs de vapeur, augmentent le taux de compression du compresseur de reprise du gaz, donc son prix et sa consommation électrique. Au final le CAPEX et les OPEX sont assez élevés.

C'est pourquoi il est possible de recycler une partie de l'effluent gazeux issu du séparateur (24) pour diluer davantage les oléfines, diminuer l'exothermicité et augmenter la pression d'opération, ce qui diminue le prix et la consommation du compresseur (26). Les autres équipements sont alors plus chers, car leur capacité et pression opératoires sont plus élevées, mais le nombre de réacteurs (223) est diminué. On peut opérer avec un seul réacteur avec un taux de recyclage suffisant, donc 3 réacteurs au total; 1 en opération, 1 en régénération et 1 « en réserve ».

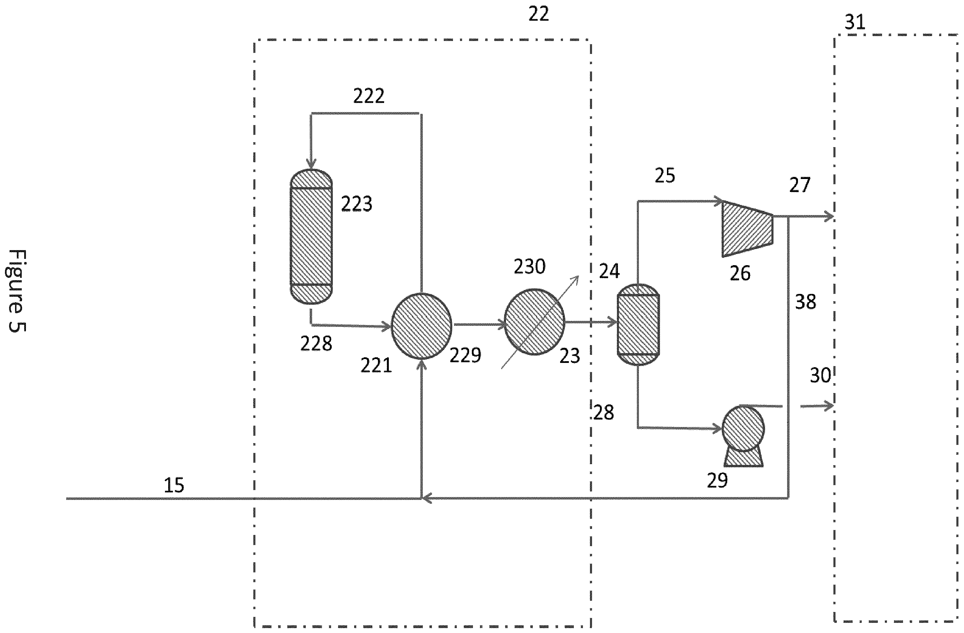

Les équipement de démarrage (notamment le four de démarrage) et de régénération (compresseur, échangeurs, séparateur, etc.) ne sont pas représentés sur les figures 4 et 5.

Description de la figure 5 (variante de la section de conversion selon l'invention) : unité de conversion de l'éthylène en propylène et autres produits d'intérêt en un seul réacteur, et recyclage d'une partie de l'effluent de l'unité de conversion.

Le flux sortie de l'absorbeur existant (6) arrive par le conduit (15), en mélange avec le recyclage arrivant par le conduit (38), vers l'échangeur charge effluent (221) où il est préchauffé jusqu'à la température d'opération et envoyé par le conduit (222) vers le réacteur (223). La réaction est très exothermique, mais avec la dilution due au recyclage, il est possible d'avoir une augmentation de température modérée de 30 à 50°C environ, donc d'opérer avec un seul réacteur. En sortie de réacteur (223), l'effluent converti est envoyé par le conduit (228) vers l'échangeur charge-effluent (221), où il est refroidi par échange indirect avec la charge, puis vers le réfrigérant (230), où le refroidissement est terminé à l'aide d'eau de refroidissement. Il est également possible d'utiliser de l'air pour refroidir. En sortie de cet échangeur, l'effluent est envoyé par le conduit (23) vers un séparateur (24), où la phase liquide est séparée de la phase gazeuse. La phase gazeuse est reprise dans le conduit (25), comprimée par le compresseur (26) et envoyée vers la section d'absorption (31).

Le liquide, soutiré par le conduit (28), est envoyée vers la section d'absorption (31) par la pompe (29) et le conduit (30).

### EXEMPLES SELON L'INVENTION

Exemple 1 : Dans ce premier exemple conforme à l'invention, on considère une unité de FCC d'une capacité de 2 MT/an, opérant pour produire un maximum de propylène (C3--).

Le procédé est opéré selon les schémas des figures 2 et 4. On opère l'unité (22) de conversion de l'éthylène en propylène au moyen de deux réacteurs l'un en réaction pendant que l'autre est en régénération.

La charge arrivant dans l'unité de conversion de l'éthylène par le conduit (15) a un débit de 16350 kg/h, avec la composition suivante (en % mole) :

| | |
|---|---|
| Azote | : 17,8 |
| Hydrogène | : 14,2 |
| Méthane | : 27,3 |
| Ethane | : 13,7 |
| Ethylène | : 13,7 |
| Propane | : 0,3 |
| Propylène | : 3,2 |
| Butane | : 0,3 |
| Butènes | : 0,4 |
| Essence | : 3,9 |
| Eau | : 0,5 |
| CO | : 1,0 |
| CO2 | : 3,8 |
| H2S, NH3, SOx, NOx sont à l'état de traces | |

La pression à l'entrée des réacteurs de conversion est de 7,8 bar absolus, ce qui permet d'avoir une pression partielle d'oléfines de 1,5 bars, et une pression à l'entrée du compresseur de 5,5 bars.

La température des réacteurs est de 550°C en entrée. On opère avec 2 réacteurs en série, avec une augmentation de la température en sortie de réacteur de 43°C dans chaque réacteur, et un refroidissement intermédiaire grâce à une génération de vapeur. On a 390 kg/h de liquide dans le séparateur.

En sortie du deuxième réacteur, la composition est la suivante (% mole) :

| | |
|---|---|
| Azote | : 18,7 |
| Hydrogène | : 15,0 |
| Méthane | : 28,7 |
| Ethane | : 14,4 |
| Ethylène | : 2,9 |
| Propane | : 0,7 |
| Propylène | : 8,5 |
| Butane | : 0,7 |
| Butènes | : 0,6 |
| Essence | : 4,5 |
| Eau | : 0,5 |
| CO | : 1,0 |
| CO2 | : 3,8 |
| H2S, NH3, SOx, NOx sont à l'état de traces | |

Globalement, la production de propylène, qui était de 21 tonnes/h sans la conversion d'éthylène, est augmentée de 1,8 tonne/h, soit 9 %, et on produit également un petit débit supplémentaire de butènes (70 kg/h) et d'essence (400 kg/h).

Exemple 2 : on considère une unité de FCC d'une capacité de 2 MT/an, opérant pour produire un maximum de propylène. On opère suivant les schémas des figures 3 et 5, c'est-à-dire avec un recyclage d'une partie du gaz sortant de l'unité de conversion (22), après compression par le compresseur (26), vers la section de conversion (22) au moyen du conduit (38).

La charge arrivant à la section de conversion de l'éthylène par le conduit (15) a un débit de 16350 kg/h comme dans l'exemple 1, avec la même composition que dans l'exemple 1.

Le recyclage sortie compresseur est de 15150 kg/h. La pression à l'entrée du réacteur est de 9 bar abs, ce qui permet d'avoir une pression partielle d'oléfines de 1,5 bar, et une pression à l'entrée du compresseur de 7,7 bar. La température en entrée du réacteur est de 550°C.

On opère avec un seul réacteur dans l'unité de conversion (22) et une augmentation de température de 44°C dans le réacteur. On a 660 kg/h de liquide dans le séparateur (24).

En sortie de réacteur, la composition est la suivante (% mole) :

| | |
|---|---|
| Azote | : 18,8 |
| Hydrogène | : 15,0 |
| Méthane | : 28,9 |
| Ethane | : 14,6 |
| Ethylène | : 1,6 |
| Propane | : 0,7 |
| Propylène | : 10,2 |
| Butane | : 0,7 |
| Butènes | : 0,4 |
| Essence | : 3,5 |
| Eau | : 0,5 |
| CO | : 1,0 |
| CO2 | : 3,9 |
| H2S, NH3, SOx, NOx sont à l'état de traces | |

Globalement, la production de propylène, qui était de 21 tonnes/h sans l'unité de conversion d'éthylène, est augmentée de 2,3 tonnes, grâce à l'unité de conversion d'éthylène en propylène (22), soit un gain de 11 %. On crée également comme effet secondaire de l'invention, un petit débit supplémentaire de butènes (30 kg/h) et d'essence (200 kg/h).

## Revendications

1. Dispositif de fractionnement de la fraction gazeuse sortant en tête de la colonne de fractionnement d'une unité de craquage catalytique, fraction contenant les GPL, l'essence légère, et un gaz résiduel dit « gaz combustible » qui contient lui-même une certaine quantité d'éthylène, dispositif comprenant :
- une première section d'absorption (6) permettant de séparer le gaz combustible (15) et le flux d'essence légère et de GPL (10), ladite première section étant existante dans une unité de craquage catalytique existante,
- une unité de conversion catalytique (22) de l'éthylène en propylène et autres produits d'intérêt, dont la charge est constituée par le gaz combustible (15), dans laquelle une grande partie de l'éthylène est convertie essentiellement en propylène et aussi en aromatiques et autres produits d'intérêt, en présence d'un catalyseur à base de zéolite.
- une nouvelle section d'absorption (31) qui admet les effluents de l'unité de conversion (22) après recompression, le propylène, butène et les fractions essences contenus dans lesdits effluents, étant récupérés dans ladite nouvelle section d'absorption (31), en utilisant de l'essence arrivant du débutaniseur existant (11),
- une seconde section d'absorption secondaire (16), qui admet le gaz (33) issu de la nouvelle section d'absorption (31),
- une section de débutanisation (35) qui admet les fractions GPL et essence (34) issus de la nouvelle section d'absorption (31), et qui produit une essence stabilisée (36), et une fraction légère GPL (37).

2. Dispositif de fractionnement de la fraction gazeuse sortant en tête de la colonne de fractionnement d'une unité de craquage catalytique selon la revendication 1, dans lequel l'unité de conversion de l'éthylène en propylène (22) comprend au moins deux réacteurs (223) et (227) séparés par un refroidissement intermédiaire de l'effluent (224) du premier réacteur (223) allant vers le second réacteur (227) au moyen d'un échangeur (225), en sortie de réacteur (227), l'effluent converti (228) étant envoyé vers un refroidissement.

3. Procédé de fractionnement de la fraction gazeuse sortant en tête de la colonne de fractionnement d'une unité de craquage catalytique, fraction contenant les GPL, l'essence légère, et un gaz résiduel dit « gaz combustible » qui contient lui-même une certaine quantité d'éthylène, comprenant les étapes suivantes :
- séparer le gaz combustible (15) et le flux d'essence légère et de GPL (10) dans une première section d'absorption (6), ladite première section étant existante dans une unité de craquage catalytique existante,
- convertir une grande partie de l'éthylène essentiellement en propylène et aussi en aromatiques et autres produits d'intérêt, en présence d'un catalyseur à base de zéolite dans une unité de conversion catalytique (22) de l'éthylène en propylène et autres produits d'intérêt, dont la charge est constituée par le gaz combustible (15).
- admettre les effluents de l'unité de conversion (22) après recompression dans une nouvelle section d'absorption (31), le propylène, butène et les fractions essences contenus dans lesdits effluents, étant récupérés dans ladite nouvelle section d'absorption (31), en utilisant de l'essence arrivant d'un débutaniseur existant (11),
- admettre le gaz (33) issu de la nouvelle section d'absorption (31) dans une seconde section d'absorption secondaire (16),
- admettre les fractions GPL et essence (34) issus de la nouvelle section d'absorption (31) dans une section de débutanisation (35) pour produire une essence stabilisée (36), et une fraction légère GPL (37).

4. Procédé selon la revendication 3, dans lequel l'unité de conversion de l'éthylène en propylène (22) comprend au moins deux réacteurs (223) et (227) séparés par un refroidissement intermédiaire de l'effluent (224) du premier réacteur (223) allant vers le second réacteur (227) au moyen d'un échangeur (225), en sortie de réacteur (227), l'effluent converti (228) étant envoyé vers un refroidissement.

5. Procédé selon la revendication 3, dans lequel une partie du flux gazeux (27) issu de l'unité de conversion de l'éthylène en propylène (23) est recyclé à l'entrée de l'unité de conversion (22) après compression dans le compresseur (26), ladite fraction recyclée pouvant varier entre 25% et 200 % du flux (27) arrivant à la nouvelle section d'adsorption (31).

6. Procédé selon la revendication 4, dans lequel les deux réacteurs de l'unité de conversion (22) fonctionnent de manière alternée l'un en réaction l'autre en régénération, le réacteur fonctionnant en régénération étant connecté à une section de régénération comprenant un échangeur charge/effluent, un échangeur de réfrigération, un séparateur permettant de condenser l'eau de réaction, et un compresseur de recirculation du gaz de régénération.

7. Procédé selon la revendication 6, dans lequel lorsque l'un des deux réacteur de l'unité de conversion (22) se trouve en régénération, ladite régénération se fait par brûlage du coke déposé sur le catalyseur en quelques heures, en injectant dans le gaz de régénération la quantité d'air adaptée, éventuellement diluée avec un inerte, par exemple de l'azote ou les gaz de combustion appauvris en oxygène, pour avoir une augmentation de température dans le réacteur limitée au maximum à 50°C.

8. Procédé selon la revendication 3, dans lequel la charge (15) de l'unité de conversion (22) arrive en mélange avec le flux recyclé (38) issu d'une partie du flux (27) vers l'échangeur charge effluent (221), dans lequel les deux flux mélangés sont préchauffé jusqu'à la température d'opération, puis envoyé vers le réacteur (223) de l'unité de conversion (22), l'effluent converti (228) étant envoyé vers l'échangeur charge-effluent (221), dans lequel il est refroidi par échange indirect avec la charge (réunion des fmlux15 et 38), puis vers le réfrigérant (230), dans lequel le refroidissement est terminé à l'aide d'eau de refroidissement, l'effluent refroidi (23) étant envoyé vers un séparateur (24), dans lequel la phase liquide est séparée de la phase gazeuse, ladite phase gazeuse (25) étant comprimée par le compresseur (26), puis envoyée vers la nouvelle section d'absorption (31), et le liquide (28), étant envoyé vers la nouvelle section d'absorption (31) par la pompe (29).

9. Procédé selon la revendication 3, dans lequel l'unité de conversion de l'éthylène en propylène (22) fonctionne avec un catalyseur à base de zéolithe aux conditions opératoires suivantes :
- température comprise entre 500 et 650°C,
- pression partielle d'éthylène comprise entre 1 et 2 bar absolus.

## Patentansprüche

1. Vorrichtung zur Fraktionierung der Gasfraktion, die am Kopf der Fraktionierungssäule einer katalytischen Krack-Einheit austritt, wobei die Fraktion LPGs, Leichtbenzin und ein als "Brenngas" bezeichnetes Restgas enthält, das seinerseits eine bestimmte Menge an Ethylen enthält, wobei die Vorrichtung umfasst:
- eine erste Absorptionssektion (6), die es gestattet, das Brenngas (15) und den Strom (10) von Leichtbenzin und LPG zu trennen, wobei die erste Sektion in einer bestehenden katalytischen Krack-Einheit vorhanden ist,
- eine Einheit (22) zur katalytischen Überführung von Ethylen in Propylen und andere Produkte von Interesse, deren Charge aus dem Brenngas (15) besteht, und in der ein großer Teil des Ethylens im Wesentlichen in Propylen und auch in aromatische Verbindungen und andere Produkte von Interesse übergeführt wird, in Anwesenheit eines Katalysators auf der Basis von Zeolith,
- eine neue Absorptionssektion (31), welche die Abflüsse der Überführungseinheit (22) nach Rekompression aufnimmt, wobei das Propylen, Buten und die Benzinfraktionen, die in den Abflüssen enthalten sind, in der neuen Absorptionssektion (31) gewonnen werden, wobei das Benzin verwendet wird, das aus dem bestehenden Debutaner (11) kommt,
- eine zweite sekundäre Absorptionssektion (16), die das Gas (33) aufnimmt, das aus der neuen Absorptionssektion (31) stammt,
- eine Entbutanisierungssektion (35), welche die LPG- und Benzinfraktionen (34) aufnimmt, die aus der neuen Absorptionssektion (31) stammen, und welche ein stabilisiertes Benzin (36) und eine Leichtfraktion LPG (37) erzeugt.

2. Vorrichtung zur Fraktionierung der Gasfraktion, die am Kopf der Fraktionierungssäule einer katalytischen Krack-Einheit austritt, nach Anspruch 1, wobei die Einheit (22) zur Überführung von Ethylen in Propylen mindestens zwei Reaktoren (223) und (227) umfasst, die durch eine Zwischenkühlung des Abflusses (224) des ersten Reaktors (223), der zu dem zweiten Reaktor (227) strömt, mittels eines Tauschers (225) am Ausgang des Reaktors (227) getrennt sind, wobei der übergeführte Abfluss (228) zu einer Kühlung geschickt wird.

3. Verfahren zur Fraktionierung der Gasfraktion, die am Kopf der Fraktionierungssäule einer katalytischen Krack-Einheit austritt, wobei die Fraktion LPGs, Leichtbenzin und ein als "Brenngas" bezeichnetes Restgas enthält, das seinerseits eine bestimmte Menge an Ethylen enthält, umfassend die folgenden Schritte:
- Trennen des Brenngases (15) und des Stroms (10) von Leichtbenzin und LPG in einer ersten Absorptionssektion (6), wobei die erste Sektion in einer bestehenden katalytischen Krack-Einheit vorhanden ist,
- Überführen eines großen Teils des Ethylens im Wesentlichen in Propylen und auch in aromatische Verbindungen und andere Produkte von Interesse, in Anwesenheit eines Katalysators auf der Basis von Zeolith, in einer Einheit (22) zur katalytischen Überführung von Ethylen in Propylen und andere Produkte von Interesse, deren Charge aus dem Brenngas (15) besteht,
- Aufnehmen der Abflüsse der Überführungseinheit (22) nach Rekompression in einer neuen Absorptionssektion (31), wobei das Propylen, Buten und die Benzinfraktionen, die in den Abflüssen enthalten sind, in der neuen Absorptionssektion (31) gewonnen werden, wobei das Benzin verwendet wird, das aus einem bestehenden Debutaner (11) kommt,
- Aufnehmen des Gases (33), das aus der neuen Absorptionssektion (31) stammt, in einer zweiten sekundären Absorptionssektion (16),
- Aufnehmen der LPG- und Benzinfraktionen (34), die aus der neuen Absorptionssektion (31) stammen, in einer Entbutanisierungssektion (35), um ein stabilisiertes Benzin (36) und eine Leichtfraktion LPG (37) zu erzeugen.

4. Verfahren nach Anspruch 3, wobei die Einheit (22) zur Überführung von Ethylen in Propylen mindestens zwei Reaktoren (223) und (227) umfasst, die durch eine Zwischenkühlung des Abflusses (224) des ersten Reaktors (223), der zu dem zweiten Reaktor (227) strömt, mittels eines Tauschers (225) am Ausgang des Reaktors (227) getrennt sind, wobei der übergeführte Abfluss (228) zu einer Kühlung geschickt wird.

5. Verfahren nach Anspruch 3, wobei ein Teil des Gasstroms (27), der aus der Einheit zur Überführung von Ethylen in Propylen (23) stammt, zum Eingang der Überführungseinheit (22), nach einer Kompression in dem Kompressor (26), recycliert wird, wobei die recyclierte Fraktion zwischen 25 % und 200 % des Stroms (27) variieren kann, der an der neuen Absorptionssektion (31) ankommt.

6. Verfahren nach Anspruch 4, wobei die beiden Reaktoren der Überführungseinheit (22) auf einander abwechselnde Weise als Regeneration betrieben werden, wobei der Reaktor, der als Regeneration betrieben wird, mit einer Regenerationssektion verbunden wird, die einen Tauscher Charge/Abfluss, einen Kühltauscher, einen Separator, der es gestattet, Wasser aus der Reaktion zu kondensieren, und einen Kompressor zur Rückführung des Regenerationsgases umfasst.

7. Verfahren nach Anspruch 6, wobei, wenn sich einer der beiden Reaktoren der Überführungseinheit (22) in der Regeneration befindet, die Regeneration durch eine Verbrennung von Koks, der auf dem Katalysator aufgebracht wird, in einigen Stunden erfolgt, indem in das Regenerationsgas die geeignete Menge an Luft injiziert wird, gegebenenfalls verdünnt mit einem Inertgas, zum Beispiel Stickstoff oder sauerstoffarmen Verbrennungsgasen, um eine Erhöhung der Temperatur in dem Reaktor zu erhalten, die maximal auf 50 °C begrenzt ist.

8. Verfahren nach Anspruch 3, wobei die Charge (15) der Überführungseinheit (22) gemischt mit dem recyclierten Strom (38), der von einem Teil des Stroms (27) stammt, in dem Tauscher (221) Charge-Abfluss ankommt, in dem die beiden gemischten Ströme bis auf eine Betriebstemperatur vorgeheizt werden, dann zu dem Reaktor (223) der Überführungseinheit (22) geschickt werden, wobei der übergeführte Abfluss (228) zu dem Tauscher (221) Charge-Abfluss geschickt wird, in dem er durch indirekten Austausch mit der Charge abgekühlt wird (Vereinigung der Ströme 15 und 38), dann zu dem Kühlmittel (230), in dem die Kühlung mit Hilfe von Kühlwasser beendet wird, wobei der abgekühlte Abfluss (23) zu einem Separator (24) geschickt wird, in dem die Flüssigphase von der Gasphase getrennt wird, wobei die Gasphase (25) von dem Kompressor (26) komprimiert wird, dann zu der neuen Absorptionssektion (31) geschickt wird, und die Flüssigkeit (28) von der Pumpe (29) zu der neuen Absorptionssektion (31) geschickt wird.

9. Verfahren nach Anspruch 3, wobei die Einheit (22) zur Überführung von Ethylen in Propylen mit einem Katalysator auf der Basis von Zeolith unter den folgenden Betriebsbedingungen betrieben wird:
- Temperatur zwischen 500 und 650 °C,
- Ethylen-Partialdruck zwischen 1 und 2 bar absolut.

## Claims

1. A device for fractionating the gaseous fraction leaving the head of the fractionation column of a catalytic cracking unit, the fraction containing LPG, light gasoline, and a residual gas termed "fuel gas" which itself contains a certain quantity of ethylene, the device comprising:
- a first absorption section (6) which can be used to separate the fuel gas (15) and the light gasoline and LPG stream (10), said section being present in an existing catalytic cracking unit,
- a unit (22) for the catalytic conversion of ethylene into propylene and other products of interest, wherein the feed is constituted by the fuel gas (15), in which a large portion of the ethylene is essentially converted into propylene and also into aromatics and other products of interest, in the presence of a catalyst based on zeolite,
- a novel absorption section (31) which admits the effluents from the conversion unit (22) after recompression, the propylene, butene and the gasoline fractions contained in said effluents being recovered in said novel absorption section (31), by using the gasoline arriving from the existing debutanizer (11),
- a second absorption section (16), which admits the gas (33) obtained from the novel absorption section (31),
- a debutanization section (35) which admits the LPG and gasoline fractions (34) obtained from the novel absorption section (31) and which produces a stabilized gasoline (36) and a light LPG fraction (37).

2. The device for fractionating the gaseous fraction leaving the head of the fractionation column of a catalytic cracking unit as claimed in claim 1, in which the unit (22) for the conversion of ethylene into propylene comprises at least two reactors (223) and (227) separated by an intermediate cooling of the effluent (224) from the first reactor (223) going towards the second reactor (227), by means of an exchanger (225) at the outlet from the reactor (227), the converted effluent (228) being sent to a chiller.

3. A process for fractionating the gaseous fraction leaving the head of the fractionation column of a catalytic cracking unit, the fraction containing LPG, light gasoline, and a residual gas termed "fuel gas" which itself contains a certain quantity of ethylene, comprising the following steps:
- separating the fuel gas (15) and the light gasoline and LPG stream (10) in a first absorption section (6), said first section being existent in an existing catalytic cracking unit,
- converting a large portion of the ethylene essentially into propylene and also into aromatics and other products of interest, in the presence of a catalyst based on zeolite in a unit (22) for the catalytic conversion of ethylene into propylene and other products of interest, wherein the feed is constituted by the fuel gas (15),
- admitting the effluents from the conversion unit (22), after recompression, into a novel absorption section (31), the propylene, butene and the gasoline fractions contained in said effluents being recovered in said novel absorption section (31), by using the gasoline arriving from an existing debutanizer (11),
- admitting the gas (33) obtained from the novel absorption section (31) into a second absorption section (16),
- admitting the LPG and gasoline fractions (34) obtained from the novel absorption section (31) into a debutanizer section (35) in order to produce a stabilized gasoline (36) and a LPG fraction (37).

4. The process as claimed in claim 3, in which the unit for the conversion of ethylene into propylene (22) comprises at least two reactors (223) and (227) separated by an intermediate cooling of the effluent (224) from the first reactor (223) going towards the second reactor (227) by means of an exchanger (225), at the outlet from the reactor (227), the converted effluent (228) being sent to a chiller.

5. The process as claimed in claim 3, in which a portion of the gas stream (27) obtained from the unit for the conversion of ethylene into propylene (23) is recycled to the inlet to the conversion unit (22) after compression in the compressor (26), said recycled fraction possibly being varied between 25% and 200% of the stream (27) arriving at the novel absorption section (31).

6. The process as claimed in claim 4, in which the two reactors of the conversion unit (22) function in alternation, one being in reaction mode and the other in regeneration mode, the reactor functioning in regeneration mode being connected to a regeneration section comprising a feed/effluent exchanger, a cooling exchanger, a separator for condensing the water of reaction, and a compressor for recirculating regeneration gas.

7. The process as claimed in claim 6 in which, when one of the two reactors of the conversion unit (22) is in regeneration mode, said regeneration is carried out by burning coke deposited on the catalyst over several hours, by injecting into the regeneration gas the appropriate quantity of air, optionally diluted with an inert gas, for example nitrogen or oxygen-depleted combustion gases, in order to produce an increase in temperature in the reactor limited to a maximum of 50°C.

8. The process as claimed in claim 3, in which the feed (15) for the conversion unit (22) arrives at the feed-effluent exchanger (221) as a mixture with the recycled stream (38) obtained from a portion of the stream (27), in which the two mixed streams are preheated to the operating temperature, then sent to the reactor (223) of the conversion unit (22), the converted effluent (228) being sent to the feed-effluent exchanger (221), in which it is cooled by indirect exchange with the feed (combination of streams 15 and 38), then towards the chiller (230) in which cooling is terminated with the aid of cooling water, the cooled effluent (23) being sent to a separator (24), in which the liquid phase is separated from the gas phase, said gas phase (25) being compressed by the compressor (26) then sent to the absorption section (31), and the liquid (28) being sent to the novel absorption section (31) via the pump (29).

9. The process as claimed in claim 3, in which the unit (22) for the conversion of ethylene into propylene functions with a catalyst based on zeolite, under the following operating conditions:
- temperature in the range 500°C to 650°C,
- a partial pressure of ethylene in the range 1 to 2 bar absolute.
